# EUROPEAN PATENT APPLICATION

(11) **EP 1 690 558 A1**
(43) Date of publication of application: **16.08.2006**
(21) Application number: 05002935.4
(22) Date of filing: 11.02.2005
(51) Int. Cl.: A61L 27/54, A61L 29/16, A61L 31/16, A61K 31/74, A61K 31/785, A61L 27/34, A61L 29/08, A61L 31/10

(54) **Device for treatment of diabetic disorders**

(71) Applicant: NOLabs AB, 252 21 Helsingborg (SE)
(72) Inventor: Peters, Tor, 8703 Erlenbach (CH)
(74) Representative: Karlsson, Leif Gunnar Börje

(57) **Abstract**

A device is provided that allows for target treatment of neuropathy, such as diabetic peripheral neuropathy or ulcers, vaso-constrictive disorders, macro-angiopathy and slow healing wounds as consequence of diabetic neuropathy. The device comprises a nitric oxide (NO) eluting polymer arranged to contact the infected area, such that a therapeutic dose of nitric oxide is eluted from said nitric oxide eluting polymer to said area.

## Description

### Field of the Invention

This invention pertains in general to the field of treatment of neuropathy, such as diabetic peripheral neuropathy, vaso-constrictive disorders, macro-angiopathy and slow healing wounds as consequence of diabetic neuropathy and impaired blood circulation due to diabetic disease or obstructed blood flow caused by other disease. More particularly the invention relates to a device for treatment of said disorders, and a process for manufacturing of said device, involving the use of nitric oxide (NO).

### Background of the Invention

Diabetes is a disorder that affects millions of people around the world. This disease results in increased risk for neuropathy and macro-angiopathy. Reasons for diabetic neuropathy are varying levels of insulin or blood sugar respectively, and high blood sugar. Diabetic neuropathy, or nerve damage, resulting from chronically high blood sugars, can be one of the most frustrating and debilitating complications of diabetes, because of the pain, discomfort and disability it can cause, and because available treatments are not uniformly successful.

Diabetic neuropathy may be divided into the three main types of neuropathy; sensory neuropathy, autonomic neuropathy, and motor neuropathy. Sensory neuropathy leads to pain, numbness, tingling in the extremities, and even the inability to feel heat, cold, pain or any other sensation in the affected areas. Autonomic neuropathy leads to impotence (in men), bladder neuropathy, diabetic diarrhea, or swollen stomach. Motor neuropathy leads to muscle weakness.

As a direct and indirect consequence of diabetic neuropathy the person suffering from this disorder may develop ulcers, so-called diabetic ulcers. These diabetic ulcers may develop as a result of small traumas on the body. Since the diabetic person, suffering from diabetic neuropathy, has a reduced, and some times even non-existent, ability to feel, these ulcers may develop very rapidly. Other factors that severely affect this matter are the reduced ability to heal and the reduced circulation in the affected area, which accompanies diabetic neuropathy. The reduced circulation in the affected area is a consequence of macro-angiopathy. Macro-angiopathy is a condition in which the blood vessels are enlarged and the arteries are hardened. These ulcers often result in amputation of the affected extremity. Macro-angiopathy is closely related to Peripheral Artery Obstructive Disease, and will therefore here on after be treated as the same.

Other malfunctions arising from diabetic neuropathy are vaso-constrictive disorders, such as Raynouds, Reyes syndrome.

Up to this point there are no good cures or treatment of neuropathy. Some medications, such as acetaminophen, aspirin, ibuprofen, amitriptyline, desipramine, and capsaicin, are on the market to liberate the diabetic person from pain, but these medicaments do not deal with the malfunction itself, only with the symptoms, and sometimes develop resistance against the active pharmaceutical substance in the medication. The person suffering from said disorders is instructed to apply these medicaments in form of creams and peroral compositions regularly during the day, rather than waiting for the pain to become severe. This is an all day process, which is very frustrating for the person suffering from said disorders. It is believed, in some circumstances, that these creams and compositions block pain signals, although they do not work for everyone. Occasionally, these creams and compositions may even worsen the pain or cause other adverse effects, such as eye or skin irritation. In the late 1980s and early 1990s aldose reductase inhibitors where introduces. Unfortunately, to date none of these drugs has proven to be sufficiently effective and adverse side effects have been a concern. Instead, the best advice the person suffering from said disorder can get, is to control and regulate his/her way of living, such as keeping blood sugar levels as close to normal as possible, exercise regularly, and make sure their weight is as close to what it should be as possible.

It is known that nitric oxide (NO) provides an alternative to conventional therapies, such as antibiotics. Nitric oxide is a highly reactive molecule that is involved in many cell functions. In fact, nitric oxide plays a crucial role in the immune system and is utilized as an effector molecule by macrophages to protect itself against a number of pathogens, such as fungi, viruses, bacteria etc., and general microbial invasion. This improvement of healing is partly caused by NO inhibiting the activation or aggregation of blood platelets, and also by NO causing a reduction of inflammatory processes at the site of an implant.

NO is also known to have an anti-pathogenic, especially an anti-viral, effect, and furthermore NO has an anti-cancerous effect, as it is cytotoxic and cytostatic in therapeutic concentrations, i.e. it has among other effects tumoricidal and bacteriocidal effects. NO has for instance cytotoxic effects on human haematological malignant cells from patients with leukaemia or lymphoma, whereby NO may be used as a chemotherapeutic agent for treating such haematological disorders, even when the cells have become resistant to conventional anti-cancer drugs. This anti-pathogenic and anti-tumour effect of NO is taken advantage of by the present invention, without having adverse effects as for instance many anti-cancer drugs.

However, due to the short half-life of NO, it has hitherto been very hard to treat viral, bacteria, virus, fungi or yeast infections with NO. This is because NO is actually toxic in high concentrations and has negative effects when applied in too large amounts to the body. NO is actually also a vasodilator, and too large amounts of NO introduced into the body will cause a complete collapse of the circulatory system. On the other hand, NO has a very short half-life of fractions of a second up to a few seconds, once it is released. Hence, administration limitations due to short half-life and toxicity of NO have been limiting factors in the use of NO in the field of anti-pathogenic and anti-cancerous treatment so far.

In recent years research has been directed to polymers with the capability of releasing nitrogen oxide when getting in contact with water. Such polymers are for example polyalkyleneimines, such as L-PEI (Linear PolyEthyleneImine) and B-PEI (Branched PolyEthyleneImine), which polymers have the advantage of being biocompatible with natural products, after the release of nitrogen oxide.

Other example for NO eluting polymers are given in US-5,770,645, wherein polymers derivatized with at least one -NOₓ group per 1200 atomic mass unit of the polymer are disclosed, X being one or two. One example is an S-nitrosylated polymer and is prepared by reacting a polythiolated polymer with a nitrosylating agent under conditions suitable for nitrosylating free thiol groups.

Akron University has developed NO-eluting L-PEI molecule that can be nano-spun onto the surface of permanently implanted medical devices such as implanted grafts, showing significant improvement of the healing process and reduced inflammation when implanting such devices. According to US-6,737,447, a coating for medical devices provides nitric oxide delivery using nanofibers of linear poly(ethylenimine)-diazeniumdiolate. Linear poly(ethylenimine)diazeniumdiolate releases nitric oxide (NO) in a controlled manner to tissues and organs to aid the healing process and to prevent injury to tissues at risk of injury. Electrospun nano-fibers of linear poly(ethylenimine) diazeniumdiolate deliver therapeutic levels of NO to the tissues surrounding a medical device while minimizing the alteration of the properties of the device. A nanofiber coating, because of the small size and large surface area per unit mass of the nanofibers, provides a much larger surface area per unit mass while minimizing changes in other properties of the device.

However, the disclosure is both silent concerning an improvement of present technology in respect of treatment of neuropathy, such as diabetic neuropathy, diabetic ulcers, vaso-constrictive disorders, and enlarged and hardened blood vessels, and the anti pathogenic potential of nitric oxide.

Hence, an improved device for therapeutic treatment and/or prevention of neuropathy, such as diabetic neuropathy, diabetic ulcers, and macro-angiopathy, which device does increase circulation in the affected area while affecting nerves positively, has a vaso-dilating effect, reduces pain and heals wounds, which device is easy to use, and cost effective, and which device does not develop resistance against the active pharmaceutical substance, and which does not cause local skin or eye irritation, pain etc, would be advantageous, and in particular a device allowing for target prevention and treatment of neuropathy, such as diabetic neuropathy, diabetic ulcers, and enlarged and hardened blood vessels, would be advantageous.

### Summary of the Invention

Accordingly, the present invention preferably seeks to mitigate, alleviate or eliminate one or more of the above-identified deficiencies in the art and disadvantages singly or in any combination and solves at least the problems mentioned above, by providing a device according to the appended patent claims.

According to one aspect of the invention, a device is provided that allows for target treatment and/or prevention of neuropathy, such as diabetic neuropathy, diabetic ulcers, vaso-constrictive disorders and macro-angiopathy. The device comprises a nitric oxide (NO) eluting polymer arranged to contact the affected area, such that a therapeutic dose of nitric oxide is eluted from said nitric oxide eluting polymer to said area.

According to another aspect of the invention, a manufacturing process for such a device is provided, wherein the process is a process for forming a device that allows for target treatment and/or prevention of neuropathy, such as diabetic neuropathy, diabetic ulcers, and macro-angiopathy. The process comprises selecting a plurality of nitric oxide eluting polymeric fibers, and deploying said nitric oxide eluting fibers in a condom/sheath, patch/dressing or tape/coating to be comprised in said device.

The present invention has at least the advantage over the prior art that it provides target exposure of an affected area to NO, whereby an increased circulation in the affected area, a vaso-dilating effect, a positive effect on nerves, pain reduction and wound healing, while not developing resistance against the active pharmaceutical substance, local skin or eye irritation, pain etc, are simultaneously obtained.

### Brief Description of the Drawings

These and other aspects, features and advantages of which the invention is capable of will be apparent and elucidated from the following description of embodiments of the present invention, reference being made to the accompanying drawings, in which
Fig. 1 is a schematic illustration of a sock according to the invention,
Fig. 2 is a schematic illustration of a tape or coating according to the invention, and
Fig. 3 is a schematic illustration of a sheath or plaster according to the invention.

### Description of Embodiments

The following description focuses on embodiments of the present invention applicable to a device, in form of a sock or tape/coating, which allows for simultaneous target treatment and/or prevention of neuropathy, such as diabetic neuropathy, diabetic ulcers, vaso-constictive disorders and macro-angiopathy, as well as a manufacturing method for the latter and a use of nitric oxide.

With regard to nitric oxide (nitrogen monoxide, NO), its physiological and pharmacological roles have attracted much attention and thus have been studied. NO is synthesized from arginine as the substrate by nitric oxide synthase (NOS). NOS is classified into a constitutive enzyme, cNOS, which is present even in the normal state of a living body and an inducible enzyme, iNOS, which is produced in a large amount in response to a certain stimulus. It is known that, as compared with the concentration of NO produced by cNOS, the concentration of NO produced by iNOS is 2 to 3 orders higher, and that iNOS produces an extremely large amount of NO.

In the case of the generation of a large amount of NO as in the case of the production by iNOS, it is known that NO reacts with active oxygen to attack exogenous microorganisms and cancer cells, but also to cause inflammation and tissue injury. On the other hand, in the case of the generation of a small amount of NO as in the case of the production by cNOS, it is considered that NO takes charge of various protective actions for a living body through cyclic GMP (cGMP), such as vasodilator action, improvement of the blood circulation, antiplatelet-aggregating action, antibacterial action, anticancer action, acceleration of the absorption at the digestive tract, renal function regulation, neurotransmitting action, erection (reproduction), learning, appetite, and the like. Heretofore, inhibitors of the enzymatic activity of NOS have been examined for the purpose of preventing inflammation and tissue injury, which are considered to be attributable to NO generated in a large amount in a living body. However, the promotion of the enzymatic activity (or expressed amount) of NOS (in particular, cNOS) has not been examined for the purpose of exhibiting various protective actions for a living body by promoting the enzymatic activity of NOS and producing NO appropriately.

In recent years research has been directed to polymers with the capability of releasing nitrogen oxide when getting in contact with water. Such polymers are for example polyalkyleneimines, such as L-PEI (Linear PolyEthyleneImine) and B-PEI (Branched PolyEthyleneImine), which polymers have the advantage of being biocompatible, after the release of nitrogen oxide.

The polymers employed in embodiments of the present invention may be manufactured by electro spinning. Electro spinning is a process by which a suspended polymer is charged. At a characteristic voltage a fine jet of polymer releases from the surface in response to the tensile forces generated by interaction by an applied electric field with the electrical charge carried by the jet. This process produces a bundle of polymer fibres, such as nano-fibres. This jet of polymer fibres may be directed to a surface to be treated.

Furthermore, US 6,382,526, US 6,520,425, and US 6,695,992 disclose processes and apparatuses for the production of such polymeric fibres. These techniques are generally based on gas stream spinning, also known within the fiber forming industry as air spinning, of liquids and/or solutions capable of forming fibers. Gas stream spinning is suited for producing devices according to certain embodiments of the invention.

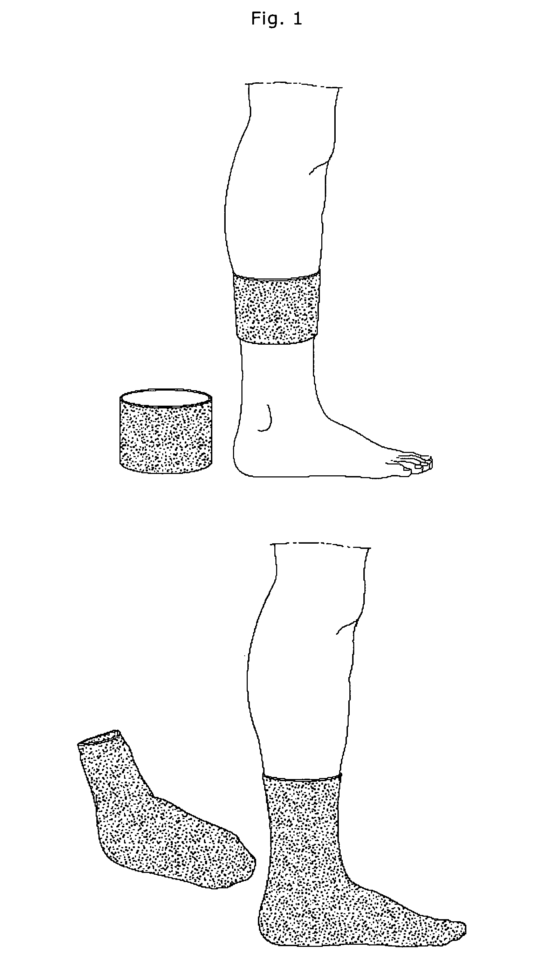

In an embodiment of the invention, according to Fig. 1, the device according to the present invention is in form of sock, manufactured of a combination of L-PEI or other NO-eluting polymer and other suitable polymers, such as polyurethane, polyvinylacetates, polylacticacids, starch, cellulose, polyhydroxyalkanoates, polyesters, polycaprolactone, polyvinylalcohol, protein based plastics, gelatine, biogradable polymers, and latex, as base material, said sock being expandable, where NO is allowed to be eluted, said sock being covered on the inside with nano-filament of NO-eluting L-PEI. The base material of the sock according to the present invention may also be cotton, polyacrylate or any other fabric used in the clothing industry, in which cases the base material is loaded with the NO-eluting polymer according to the invention. This embodiment provides an easy to use sock, which is applied on the affected area in the same way as normal clothing.

In another embodiment of the present invention the sock is covered on the inside with nano-filament of any other suitable polymer, according to above. Such polymers are for example other polyalkyleneimines, such as B-PEI (Branched PolyEthyleneImine), which polymers have the advantage of being biocompatible, after the release of nitrogen oxide.

Other example for NO eluting polymers are given in US-5,770,645, wherein polymers derivatized with at least one -NOX group per 1200 atomic mass unit of the polymer are disclosed, X being one or two. One example is an S-nitrosylated polymer and is prepared by reacting a polythiolated polymer with a nitrosylating agent under conditions suitable for nitrosylating free thiol groups.

Akron University has developed NO-eluting L-PEI molecule that can be nano-spun onto the surface of permanently implanted medical devices such as implanted grafts, showing significant improvement of the healing process and reduced inflammation when implanting such devices. According to US-6,737,447, a coating for medical devices provides nitric oxide delivery using nanofibers of linear poly(ethylenimine)-diazeniumdiolate. Linear poly(ethylenimine)diazeniumdiolate releases nitric oxide (NO) in a controlled manner.

Thus, it is most preferable that the nano-spun fibres in the NO-eluting sock according to the present embodiment of the present invention comprise L-PEI. Also nano-filaments to be woven into the sock are suitably produced from L-PEI and loaded with NO for release thereof at use.

This sock may be in any suitable size, such as a suitable size for covering any body part to be treated, such as a foot or individual toes, a calf, a thigh, the whole or a part of an abdomen, a neck, the whole or a part of a head, a shoulder, an upper arm, a forearm, a hand or individual fingers. These sizes may for example vary from small, medium, and large sized socks, depending on the size of the person to be treated.

When the NO-eluting sock according to the present embodiment of the invention is applied on the area to be treated, according to Fig. 1, and gets in contact with the moisture, in form of secreted sweat, the NO-eluting sock starts to release NO to the area to be treated.

In another embodiment of the present invention the sock according to the present invention is covered on the inside with NO-eluting nano-particles, or micro-spheres. These nano-particles, or micro-spheres, may be formed from the NO-eluting polymers according to the present invention, encapsulated in any suitable material, such as polyvinylacetates, polylacticacids, starch, cellulose, polyhydroxyalkanoates, polyesters, polycaprolactone, polyvinylalcohol, protein based plastics, gelatine, biogradable polymers, and other soluble plastics. When the nano-particles, or micro-spheres, according to this embodiment, gets in contact with the secreted moisture, in form of sweat, on the inside of the sock, they start to elute NO on the area to be treated.

In yet another embodiment of the present invention the sock contains a small water bag or sealed water sponge. This water bag or sealed water sponge is used to activate the elution of NO from the NO-eluting polymer, nano-particles, and/or micro-spheres. Persons that not easily sweat may be helped by the use of this embodiment.

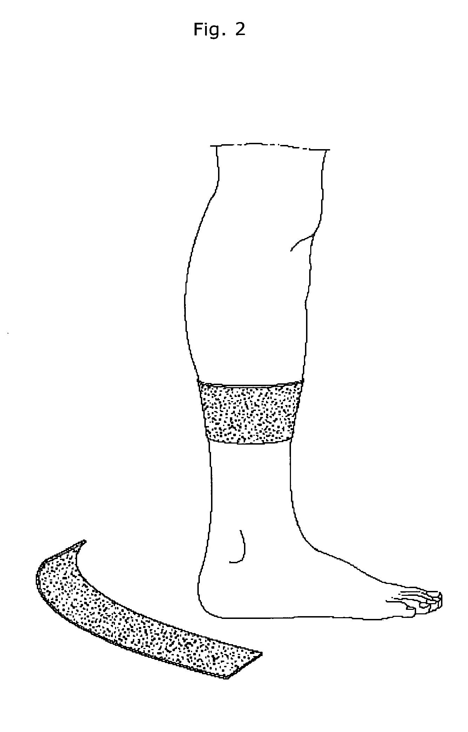

In still another embodiment of the device according to the present invention, it may be manufactured in the form of a polyurethane, or polyethylene, tape or coating, according to Fig. 2. This polyurethane tape or coating may easily be wrapped around the body part to be treated. At least the side facing the body part may be covered with NO-eluting nano-particles, micro-spheres, or nano-filament of NO-eluting L-PEI. When these particles or filaments get in contact with the moisture, in form of sweat, on the inside of the tape or coating, the elution of NO starts.

This embodiment makes it possible to obtain a device that may be applied on locations that are difficult to get at with a sock, such as in between the toes or fingers, the groin, the armpit etc.

In other embodiments of the invention, the tape or coating may be manufactured by any other suitable material, such as rubbers and plastics, polyvinylacetates, polylacticacids, starch, cellulose, polyhydroxyalkanoates, polyesters, polycaprolactone, polyvinylalcohol, protein based plastics, gelatine, biogradable polymers, and other soluble plastics.

In another embodiment these nano-particles, or micro-spheres, may be integrated in a soluble film that disintegrates on the inside of the sock or tape/coating according to the present invention, in order to elute NO at the area of interest when the soluble film gets in contact with the moisture, in form of sweat or from the water bag or sealed water sponge, on the area to be treated.

When placed on an area to be treated the device according to the present invention provides target treatment and/or prevention of neuropathy, such as diabetic neuropathy, diabetic ulcers, and macro-angiopathy.

In another embodiment of the present invention the device only allows NO-elution in one direction. In this kind of embodiment one side of the sock or tape/coating is non-permeable to NO. This may be accomplished by applying a material on one side of the sock or tape/coating that is not permeable to NO. Such materials may be chosen from the group comprising common plastics, such as polyethylene, polyurethane, polyesters, polyamides, polyethers, polycarbonates, polyacrylonitrile, polystyrene, polypropylene, poly(acrylic acid) etc.

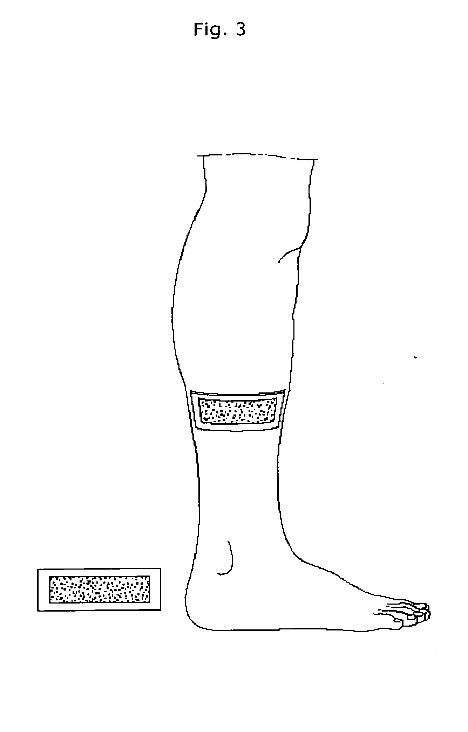

In another embodiment of the present invention, the device is in form of polyurethane or polyethylene sheaths or plasters, pads or dressings according to Fig. 3, coated with the NO-eluting polymer according to the present invention. The plaster or sheath may be applied on the area to be treated with the aid of an adhering material, such as glue etc. This embodiment has the advantage of being applicable on smaller areas or wounds, when there is no need, or if the person to be treated finds it unpleasant, to cover the whole body part with a sock or coating/tape. It is also an advantage that the device in form of a plaster or sheath or pad or dressing needs lesser amount of material, thus lower manufacturing cost.

The devices according to the embodiments may also be covered with a powder manufactured from nano-fibres of NO-eluting polymer, such as L-PEI.

In yet another embodiment of the present invention the NO-eluting device is acting as a booster for drug eluting patches, e.g. pharmaceuticals, vitamins, nicotin, nitroglycerin etc. This embodiment presents a device with the advantage of combining two therapeutic treatments, of significant value, in one treatment. Hence, a synergetic effect may be achieved by such devices when NO that is eluted from the device. NO has a vasodilatory effect on the region where the device having the combination compound actuates. Vasodilated tissue is more susceptible to certain medications and thus more easily treated by the medical preparations and still NO has in addition to that the anti-inflamatory, anti-bacterial etc. effect. Hence, an unexpected surprisingly effective treatment is provided.

In another embodiment of the device according to the present invention the fibres, nano-particles, or micro-spheres may be integrated in a gel, that may either be in a smearing or compressed structure. The elution of NO may then be initiated by applying a water soaked patch on said gel. The fibres, nano-particles, or micro-spheres may also be integrated in a hydrogel, which is mixed directly before use. This embodiment has the advantage of being able to penetrate pockets and corners in the skin for closer elution of NO on the area to be treated.

In still another embodiment the device of the present invention is in form of a cream or foam.

The device according to the present invention elutes nitric oxide (NO) from said eluting polymer in a therapeutic dose, such as between 1 to 100 ppm, such as 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90 91, 92, 93, 94, 95, 96, 97, 98, 99, or 100 ppm.

In the embodiments of the present invention it may be suitable to control or regulate the time span of NO release from the device according to the invention. This may be accomplished by integrating other polymers or materials in said device. These polymers or materials may be chosen from any suitable material or polymer, such as polyvinylacetates, polylacticacids, starch, cellulose, polyhydroxyalkanoates, polyesters, polycaprolactone, polyvinylalcohol, protein based plastics, gelatine, biogradable polymers, and other soluble plastics.

The NO-eluting polymers in the devices according to the present invention may be combined with silver, such as hydroactivated silver. The integration of silver in the devices according to the present invention gives the healing process an extra boost. Preferably the silver is releasable from the devices in the form of silver ions.

The device according to the present invention may be manufactured by, for example electro spinning of L-PEI. L-PEI is the charged at a characteristic voltage, and a fine jet of L-PEI releases as a bundle of L-PEI polymer fibres. This jet of polymer fibres may be directed to a surface to be treated. The surface to be treated may for example be any suitable material in respect of a device according to the present invention. The electro spun fibres of L-PEI then attach on said material and form a coating/layer of L-PEI on the device according to the invention.

The basic material of the device according to the present invention may be polyesters, polyamides, polyethers, polyurethanes, polycarbonates, polyvinylacetates, polylacticacids, starch, cellulose, polyhydroxyalkanoates, polyesters, polycaprolactone, polyvinylalcohol, cotton, polypropylene, polyacrylonitrile, polystyrene, poly(acrylic acid), protein based plastics, gelatine, and other biocompatible polymers. The NO-eluting polymer may be integrated in, spun together with, or spun on top of, any of these materials in all of the embodiments of the present invention.

It is of course possible to electro spin the other NO-eluting polymers, according to above, on the device according to the invention while still being inside the scope of the present invention.

In one embodiment the NO-eluting polymers employed in the devices according to the present invention are electro spun in such way that pure NO-eluting polymer fibres may be obtained.

Gas stream spinning, or air spinning, of said NO-eluting polymers onto the device according to the present invention is also within the scope of the present invention.

The manufacturing process according to the present invention presents the advantages of large contact surface of the NO-eluting polymer fibres or micro particles with the area to be treated, effective use of NO-eluting polymer, and a cost effective way of producing the device according to the present invention.

Hereinafter, some potential uses of the present invention are described:
A method of therapeutically treating and/or preventing neuropathy and ulcers at a treatment side of a body, by means of a device that comprises a nitric oxide (NO) eluting polymer configured for eluting a therapeutic dosage of nitrogen oxide (NO) when used for said treatment, comprising exposing said treatment site of said infection in or on a body to said nitric oxide when said polymer in use elutes nitrogen oxide (NO) by eluting a therapeutic dose of nitric oxide from said nitric oxide eluting polymer to said treatment site.

The method according to the above, wherein said site is an extremity of a body, and wherein said method comprises applying a condom/sheath, sock, patch/pad/dressing, and tape/coating to said extremity for said exposure.

Use of nitric oxide (NO) in a therapeutic dose for therapeutically treating and/or preventing neuropathy and ulcers at a treatment side of a body.

The invention may be implemented in any suitable form. The elements and components of the embodiments according to the invention may be physically, functionally, and logically implemented in any suitable way. Indeed, the functionality may be implemented in a single unit, in a plurality of units, or as part of other functional units.

Although the present invention has been described above with reference to specific embodiments, it is not intended to be limited to the specific form set forth herein. Rather, the invention is limited only by the accompanying claims and, other embodiments than the specific above are equally possible within the scope of these appended claims.

In the claims, the term "comprises/comprising" does not exclude the presence of other elements or steps. Furthermore, although individually listed, a plurality of means, elements or method steps may be implemented. Additionally, although individual features may be included in different claims, these may possibly advantageously be combined, and the inclusion in different claims does not imply that a combination of features is not feasible and/or advantageous. In addition, singular references do not exclude a plurality. The terms "a", "an", "first", "second" etc do not preclude a plurality. Reference signs in the claims are provided merely as a clarifying example and shall not be construed as limiting the scope of the claims in any way.

## Claims

1. A device configured for therapeutic target treatment and/or prevention of neuropathy and ulcers at a target side of a body, wherein
said device comprises a nitric oxide (NO) eluting polymer configured for eluting a therapeutic dosage of nitrogen oxide (NO) when used for said treatment and/or prevention, and
wherein said device is configured for exposing said target site in or on a body to said nitric oxide when said polymer in use elutes nitrogen oxide (NO).

2. Device according to claim 1, wherein said polymer is selected from the group consisting of polyalkyleneimines, S-nitrosylated polymer, and poly(alkylenimine)diazeniumdiolates, or any combination these.

3. Device according to claim 1, wherein said polymer is L-PEI (linear polyethyleneimine), loaded with nitric oxide(NO), arranged for release of the nitric oxide (NO) at said target site in or on a body for treatment of or prevention of neuropathy at the site.

4. Device according to claim 1, wherein said device has a form selected from the group consisting of a sock, a tape/coating, a patch or dressing and a plaster, configured for therapeutic target treatment and/or prevention of diabetic neuropathy, macro-angiopathy, diabetic ulcers, and/or vaso-constrictive disorders at said treatment site.

5. Device according to claim 8, wherein said sock, tape/coating, or plaster is manufactured from polyurethane or polyethylene, and said sock, tape/coating, or a plaster includes said nitric oxide (NO) eluting polymer configured for in use eluting said nitric oxide (NO) to said target site.

6. Device according to any of claims 1 to 5, including a water bag or sealed water sponge.

7. Device according to claim 1, wherein said device is partly disintegrable when subjected to moisture or water.

8. Device according to claim 1, wherein said polymer comprises silver, configured exposure of said target site.

9. Device according to claim 1, wherein said polymer is configured to act as a booster for drug eluting patches when used.

10. Device according to claim 1, wherein said polymer is in form of nano-particles or micro-spheres.

11. Device according to claim 10, wherein said nano-particles, or micro-spheres, are encapsulated in suitable material, such as polyvinylacetates, polylacticacids, starch, cellulose, polyhydroxyalkanoates, polyesters, polycaprolactone, polyvinylalcohol, protein based plastics, and/or gelatine.

12. Device according to claim 10, wherein said nano-particles, or micro-spheres, are integrated in a gel, hydrogel, foam or cream.

13. Device according to claim 1, wherein said device comprises a material which regulates or controls NO-elution, selected from the group consisting of polyvinylacetates, polylacticacids, starch, cellulose, polyhydroxyalkanoates, polyesters, polycaprolactone, polyvinylalcohol, protein based plastics, gelatine, and/or biogradable polymers.

14. Device according to claim 1, wherein said NO-eluting polymer is applied on, or integrated with, a material selected from the group consisting of polyesters, polyamides, polyethers, polyurethanes, polycarbonates, polyvinylacetates, polylacticacids, starch, cellulose, polyhydroxyalkanoates, polyesters, polycaprolactone, polyvinylalcohol, cotton, polypropylene, polyacrylonitrile, polystyrene, poly(acrylic acid), protein based plastics, and gelatine, or combinations thereof.

15. A manufacturing process for a device configured for therapeutic target treatment and/or prevention of neuropathy and ulcers at a target side of a body according to claim 1, comprising:
selecting a plurality of nitric oxide eluting polymeric particles, preferably nano fibres, nano particles or micro spheres, and
deploying said nitric oxide eluting particles into a suitable form, or as a coating onto a carrier, to form said device,
wherein said deploying comprises electro, air, or gas stream spinning of said fibres.

16. Use of a nitric oxide (NO) eluting polymer for the manufacture of a device for the treatment of neuropathy and ulcers, including diabetic peripheral neuropathy, macro-angiopathy, diabetic ulcers, and/or vaso-constrictive disorders, wherein
nitric oxide is loaded to said device elutes nitric oxide (NO) from said eluting polymer in a therapeutic dose when used at a target side of a body.

17. Use according to claim 16, wherein said therapeutic dose is 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90 91, 92, 93, 94, 95, 96, 97, 98, 99, or 100 ppm.
